# EUROPÄISCHE PATENTSCHRIFT

(11) **EP 0 550 509 B1**
(45) Veröffentlichungstag und Bekanntmachung des Hinweises auf die Patenterteilung: **05.06.1996**
(21) Anmeldenummer: 91916553.0
(22) Anmeldetag: 14.09.1991
(51) Int. Cl.: C07H 15/04, B01D 61/14

(54) **VERFAHREN ZUR HERSTELLUNG GEREINIGTER GLYCOLIPIDE DURCH MEMBRANTRENNVERFAHREN**
PROCESS FOR THE PRODUCTION OF PURIFIED GLYCOLIPIDS BY THE DIAPHRAGM SEPARATION METHOD
PROCEDE POUR LA FABRICATION DE GLYCOLIPIDES PURIFIES PAR UN PROCEDE DE SEPARATION A MEMBRANE

(30) Priorität: 25.09.1990 DE 4030264
(43) Veröffentlichungstag der Anmeldung: 14.07.1993
(73) Patentinhaber: HOECHST AKTIENGESELLSCHAFT, D-65926 Frankfurt am Main (DE)
(72) Erfinder: BUCHHOLZ, Rainer, D-1000 Berlin (DE); Fricke, Ulrich, D-6251 Selters 1 (DE); MIXICH, Johann, D-6233 Kelkheim (DE)
(86) Internationale Anmeldenummer: EP9101756
(87) Internationale Veröffentlichungsnummer: WO9205183

(56) Entgegenhaltungen:
- EP-A- 0 153 634
- JOURNAL OF CHEMICAL TECHNOLOGY & BIOTECHNOLOGY, Band 47, Nr. 1, 1990; C.N. MULLIGAN et al., Seiten 23-29
- TENSIDE SURFACTANTS DETERGENTS, Band 25, Nr. 4, 1988; M.P. BOSCH et al., Seiten 208-211

## Beschreibung

Glycolipide sind aus einem Fettsäure- und einem Zuckerrest zusammengesetzt. Eines der bekanntesten Glycolipide ist das Trehaloselipid (Suzuki et al, 1969, Agric. Biol. Chem., 33, 1619-1627). Es besteht aus dem Disaccharid Trehalose und zwei β-Hydroxy-α-verzweigten Fettsäuren (Corynomycolsäure) die mit Hydroxy-Gruppen des Zuckers verestert sind. Glycolipide, die Rhamnose und β-Hydroxyfettsäuren enthalten, werden in EP 0 153 634 beschrieben. Das erste Rhamnolipid, dessen Struktur bestimmt wurde, besteht aus zwei Molekülen L-Rhamnose und zwei Molekülen β-Hydroxydecansäure (Ewards und Hayashi, 1965, Arch. Biochem. Biophys., 111, 415-421).

Die bisher bekannten Glycolipide haben je nach Zucker- und Fettsäureanteil ein Molekulargewicht zwischen 250 und 2000. Sie werden beispielsweise von Bakterien ins umgebende Nährmedium abgegeben und können als Mischung von mehreren verschiedenen Glycolipiden angehäuft werden (EP 0 153 634; Advances in Appl. Microbiol. 1980, 229-259).

Glycolipide können beispielsweise als Emulgatoren, Biotenside oder Stabilisatoren für Emulsionen verwendet werden.

Die Reinigungsverfahren von Glycolipiden beruhen auf Extraktions-, Kristallisations- und Chromatographieprozessen (EP 0 282 942; US 4 812 272). Bei diesen Verfahren fallen jedoch große Mengen Lösemittel an. Mulligan und Gibbs (J. Chem. Tech. Biotechnol, 1990, 47, 23-29) beschreiben ein Ultrafiltrationsverfahren zur Reinigung von Biotensiden, wie Surfactin und Rhamnolipiden. Das dort beschriebene Rückhaltevermögen der Membranen nimmt jedoch mit zunehmender Porengröße ab. Dies führt bei Membranen mit einer Trenngrenze von 30 000 zu Verlusten an Glycolipiden von mehr als 77 %.

Überraschend wurde nun gefunden, daß Glycolipide von einer Membran, die eine Trenngrenze für Moleküle mit einem Molekulargewicht von mehr als 30 000 hat, bei saurem pH annähernd vollständig zurückgehalten werden.

Die Erfindung betrifft somit ein Verfahren zur Herstellung gereinigter Glycolipide, das dadurch gekennzeichnet ist, daß die Reinigung mit Hilfe eines Membrantrennverfahrens bei saurem pH-Wert mit einer Membran, die eine Trenngrenze für Moleküle mit einem Molekulargewicht von mehr als 30 000 hat, durchgeführt wird.

Die Erfindung wird im folgenden detailliert beschrieben, insbesondere in ihren bevorzugten Ausführungsformen.

Glycolipide können in Pflanzen oder Bakterien vorkommen (Lehninger, Biochemistry, 1977; Syldakt et al. Z. Naturforschung, 40 c, 1985, 51 - 67). Für die Herstellung der Glycolipide kommt bevorzugt die Fermentationen in Betracht. Dazu werden die Mikroorganismen in an sich bekannter Weise kultiviert. Die in das Kulturmedium ausgeschiedenen Glycolipide werden bespielsweise nach beendeter Fermentation, mit Hilfe des erfindungsgemäßen Membrantrennverfahrens, vorzugsweise Ultrafiltration, gereinigt. Die Ultrafiltration kann z. B. in Platten-, Rohr-, Kapillarrohr-, Wickelmembran- oder Hohlfaserapparaturen durchgeführt werden, die Membranen mit einer Trenngrenze von 30 000 bis 300 000 enthalten. Es werden bevorzugt Membranen mit einer Trenngrenze von 100.000 bis 200 000 eingesetzt. Die Trenngrenze bezieht sich auf das Molekulargewicht der zurückzuhaltenden Moleküle und bedeutet, daß Stoffe mit einem Molekulargewicht größer als dem genannten Wert zurückgehalten werden. Als Membranmaterial können z. B. Polysulfone, Polyamide oder Celluloseacetate verwendet werden. Die Glycolipide werden von der Membran zurückgehalten und können nach der Reinigung weiterverarbeitet werden.

Das erfindungsgemäße Membrantrennverfahren wird bei saurem pH-Wert, bevorzugt bei einem pH von 1 bis 5, besonders bevorzugt bei einem pH-Wert von 2 bis 4, durchgeführt. Die Einstellung des pH-Wertes erfolgt, wenn es notwendig ist, mit einer Säure oder Lauge. Es können beispielsweise Salzsäure, Schwefelsäure, Essigsäure, Citronensäure, Natriumhydroxid oder Natriumcarbonat verwendet werden.

Das erfindungsgemäße Verfahren wird vorteilhaft für die Reingigung von Rhamnolipiden eingesetzt. Die Reinigung von α-L-Rhamnopyranosyl-β-hydroxydecansäure,
2-O-α-L-Rhamnopyranosyl-α-L-rhamnopyranosyl-β-hydroxydecansäure,
2-O-α-L-Rhamnopyranosyl-α-L-rhamnopyranosyl-β-hydroxydecanoyl-β-hydroxydecansäure oder α-L-Rhamnopyranosyl-β-hydroxydecanoyl-β-hydroxydecansäure, ist besonders bevorzugt.

Die Reinigung der Glycolipide mit einer Ultrafiltrationsanlage kann chargenweise oder kontinuierlich durchgeführt werden. Dabei wird die Temperatur der Filtrationslösung zwischen 0°C und 70°C, bevorzugt zwischen 18°C und 40°C, gehalten.

Die Überströmgeschwindigkeit der Flüssigkeit über der Membran wird zwischen 2 und 7 m/s, bevorzugt zwischen 5 und 6 m/s, gehalten. Der Flüssigkeitsüberdruck am Eingang der Filtrationsanlage beträgt zwischen 3 und 6 bar. Der Druckverlust über die Filtrationsstrecke liegt zwischen 0,5 und 3,5 bar.

Das erfindungsgemäße Membrantrennverfahren kann natürlich nicht nur zur Reinigung sondern auch zur Aufkonzentrierung der Glycolipide verwendet werden.

### Beispiel 1

Pseudomonas spec. DSM 2874 wurde gemäß deutscher Patentschrift 3 405 664 unter aeroben Bedingungen in einer Nährlösung mit Paraffin S (89 % Tetradecan, 9 % Pentadecan) als Kohlenstoffquelle gezüchtet. Die Fermentation erfolgte bei einem pH-Wert 6,6 und 7,2 sowie bei 30°C und einer Belüftungsrate von 0,2 V/V/m. Die Fermentation wurde nach 7 Tagen beendet. Die Fermentationslösung enthielt ein Rhamnoselipidgemisch mit einem L-Rhamnoseanteil von 2,5 g/l.

### Beispiel 2

Die aus Beispiel 1 gewonnene Fermentationslösung wurde mit Schwefelsäure auf pH 3 eingestellt und anschließend mit einer Rohrmodulultrafiltrationsanlage (Böttcher, Österreich) aufkonzentriert. Es wurden folgende Prozeßparameter eingehalten:

| | |
|---|---|
| Eingangsdruck : | 5 bar |
| Ausgangsdruck : | 1,5 bar |
| Temperatur : | 35° C |
| Überströmgeschwindigkeit : | 5 m/s |
| Filtrationsleistung : | 200 l/h |
| Konzentrierungsfaktor : | 3,9 |
| Anfangsvolumen : | 18,6 m³ |
| Endvolumen : | 4,8 m³ |

Nach der Aufkonzentrierung enthielt das Konzentrat ein Rhammoselipidgemisch mit einem L-Rhamnoseanteil von 9,75 g/l.

### Beispiel 3

Die aus Beispiel 2 erhaltene aufkonzentrierte Fermentationslösung wurde durch Diafiltration bei pH 3 und konstantem Volumen mit Wasser entsalzt. Dazu wurde eine Rohrmodulultrafiltrationsanlage (Böttcher, Österreich) verwendet. Die Polysulfonmembran UF-PS-100, Fa. Kalle-Albert, Wiesbaden, hatte eine Trenngrenze von 100 000. Es wurden folgende Prozeßparameter eingehalten:

| | |
|---|---|
| Eingangsdruck : | 5 bar |
| Ausgangsdruck : | 1,5 bar |
| Temperatur : | 35°C |
| Überströmgeschwindigkeit : | 5 m/s |
| Filtrationsleistung : | 59 l/m²h |
| Volumen der Lösung : | 4,8 m³ |

Die Fermentationsbrühe hatte vor der Entsalzung eine Leitfähigkeit von 24,5 mS cm⁻¹ und danach 2,9 mS cm⁻¹. Das Permeat enthielt 0,1 g/l L-Rhamnose.

### Beispiel 4

Vergleichend wurde Rhamnoselipid enthaltende Fermentationslösung über Membranen mit einer Trenngröße von 10 000 bzw. 100 000 gegeben:

| Prozeßparameter: | |
|---|---|
| Überströmung | 0,3 m³/h |
| Ausgangsdruck | 4 bar |
| Temperatur | 32°C |
| Membranfläche | 0,45 m² |
| pH | 3 |

| Membrantypen | Permeat | Flux l/m²h |
|---|---|---|
| Polysulfon 10 000 D | klar | 0,8 |
| Polysulfon 100 000 D | klar | 50,0 |

In beiden Fällen betrug der Rückhalt an Rhamnolipid 99 %.

## Patentansprüche

1. Verfahren zur Herstellung gereinigter Glycolipide dadurch gekennzeichnet, daß die Reinigung mit Hilfe eines Membrantrennverfahrens bei saurem pH-Wert mit einer Membran, die eine Trenngrenze für Moleküle mit einem Molekulargewicht von mehr als 30 000 hat, durchgeführt wird.

2. Verfahren nach Anspruch 1, dadurch gekennzeichnet, daß Rhamnolipide gereinigt werden.

3. Verfahren nach Anspruch 1, dadurch gekennzeichnet, daß α-L-Rhamnopyranosyl-β-hydroxydecansäure,
2-O-α-L-Rhamnopyranoyl-α-L-rhamnopyranosyl-β-hydroxydecansäure,
2-O-α-L-Rhamnopyranoyl-α-L-rhamnopyranosyl-β-hydroxydecanoyl-β-hydroxydecansäure oder α-L-Rhamnopyranosyl-β-haydroxydecanoyl-β-hydroxydecansäure gereinigt werden.

4. Verfahren nach einem oder mehreren der Ansprüche 1 bis 3, dadurch gekennzeichnet, daß als Membrantrennverfahren die Ultrafiltration dient.

5. Verfahren nach einem oder mehreren der Ansprüche 1 bis 4, dadurch gekennzeichnet, daß eine Ultrafiltrationsmembran mit einer Trenngrenze von 30 000 bis 300 000 eingesetzt wird.

6. Verfahren nach Anspruch 1 bis 5, dadurch gekennzeichnet, daß eine Ultrafiltrationsmembranen mit einer Trenngrenze von 100 000 bis 200 000 eingesetzt wird.

7. Verfahren nach einem oder mehreren der Ansprüche 1 bis 6, dadurch gekennzeichnet, daß das Verfahren bei einem pH-Wert von 1 bis 5 durchgeführt wird.

8. Verfahren nach Anspruch 7, dadurch gekennzeichnet, daß das Verfahren bei einem pH-Wert von 2 bis 4 durchgeführt wird.

9. Verfahren nach einem oder mehreren der Ansprüche 1 bis 8, dadurch gekennzeichnet, daß bei einer Temperatur von 0°C bis 70°C gearbeitet wird.

## Claims

1. A process for preparing purified glycolipids, which comprises carrying out the purification using a membrane separation process at acid pH with a membrane which has a cut-off size for molecules with a molecular weight of more than 30,000.

2. The process as claimed in claim 1, wherein rhamnolipids are purified.

3. The process as claimed in claim 1, wherein α-L-rhamnopyranosyl-β-hydroxydecanoic acid, 2-O-α-L-rhamnopyranosyl-α-L-rhamnopyranosyl-β-hydroxydecanoic acid, 2-O-α-L-rhamnopyranosyl-α-L-rhamnopyranosyl-β-hydroxydecanoyl-β-hydroxydecanoic acid or α-L-rhamnopyranosyl-β-hydroxydecanoyl-β-hydroxydecanoic acid are purified.

4. The process as claimed in one or more of claims 1 to 3, wherein ultrafiltration serves as the membrane separation process.

5. The process as claimed in one or more of claims 1 to 4, wherein an ultrafiltration membrane with a cut-off size of 30,000 to 300,000 is employed.

6. The process as claimed in claim 1 to 5, wherein an ultrafiltration membrane with a cut-off size of 100,000 to 200,000 is employed.

7. The process as claimed in one or more of claims 1 to 6, wherein the process is carried out at a pH of 1 to 5.

8. The process as claimed in claim 7, wherein the process is carried out at a pH of 2 to 4.

9. The process as claimed in one or more of claims 1 to 8, wherein it is carried out at a temperature of 0°C to 70°C.

## Revendications

1. Procédé de préparation de glycolipides purifiés, caractérisé en ce que la purification est effectuée à l'aide d'un procédé de séparation sur membrane, à pH acide, avec une membrane qui présente un seuil de coupure pour les molécules ayant une masse moléculaire de plus de 30 000.

2. Procédé selon la revendication 1, caractérisé en ce que l'on purifie des rhamnolipides.

3. Procédé selon la revendication 1, caractérisé en ce que l'on purifie l'acide α-L-rhamnopyrannosyl-β-hydroxydécanoïque, l'acide 2-O-α-L-rhamnopyrannosyl-α-L-rhamnopyrannosyl-β-hydroxydécanoïque, l'acide 2-O-α-L-rhamnopyrannosyl-α-L-rhamnopyrannosyl-β-hydroxydécanoyl-β-hydroxydécanoïque ou l'acide α-L-rhamnopyrannosyl-β-hydroxydécanoyl-β-hydroxydécanoïque.

4. Procédé selon une ou plusieurs des revendications 1 à 3, caractérisé en ce que, comme procédé de séparation sur membrane, on utilise l'ultrafiltration.

5. Procédé selon une ou plusieurs des revendications 1 à 4, caractérisé en ce que l'on utilise une membrane d'ultrafiltration ayant un seuil de coupure de 30 000 à 300 000.

6. Procédé selon l'une des revendications 1 à 5, caractérisé en ce que l'on utilise une membrane d'ultrafiltration ayant un seuil de coupure de 100 000 à 200 000.

7. Procédé selon une ou plusieurs des revendications 1 à 6, caractérisé en ce le procédé est effectué à un pH de 1 à 5.

8. Procédé selon la revendication 7, caractérisé en ce que le procédé est effectué à un pH de 2 à 4.

9. Procédé selon une ou plusieurs des revendications 1 à 8, caractérisé en ce que l'on opère à une température de 0 à 70°C.
